# DEMANDE DE BREVET EUROPEEN

(11) **EP 1 709 996 A1**
(43) Date de publication de la demande: **11.10.2006**
(21) Numéro de dépôt: 05290774.8
(22) Date de dépôt: 07.04.2005
(51) Int. Cl.: A61Q 5/02, A61K 8/02, A61K 8/31, A61K 8/34, A61K 8/60, A61K 8/81

(54) **Composition aqueuse à base d'alcool, de tensioactif et d'agent solubilisant, article imprégné de cette composition et procédé de nettoyage des cheveux**

(71) Demandeur: L'ORÉAL, 75008 Paris (FR)
(72) Inventeur: Decoster, Sandrine, 95210 Saint Gratien (FR); Masse, Virginie, 92110 Clichy (FR)
(74) Mandataire: Le Blainvaux Bellegarde, Françoise

(57) **Abrégé**

La présente invention a pour objet une composition aqueuse contenant un tensioactif particulier, un monoalcool en C2-C4, et un agent solubilisant les composés liposolubles. Elle a également pour objet un article cosmétique imprégné de cette composition et l'utilisation de cette composition pour le nettoyage des cheveux, et un procédé de nettoyage de ces derniers utilisant cette composition ou cet article.

## Description

La présente invention a pour objet une composition capillaire destinée au nettoyage des cheveux comprenant dans un milieu aqueux cosmétiquement acceptable au moins un monoalcool en C2-C4, au moins un agent tensioactif, et au moins un agent solubilisant des composés liposolubles et un procédé nettoyage de ces derniers utilisant cette composition.

Les chevelures ont tendance à perdre certaines de leurs qualités sous l'action de facteurs tels que le regraissage naturels, la sueur, l'élimination de squames, la pollution, l'humidité et autres. Ces facteurs nuisent à l'aspect visuel et au toucher des cheveux. Ainsi, le regraissage (et éventuellement la pollution) alourdit les cheveux, lesquels ont tendance à se mettre en paquets. Les cheveux sont alors difficiles à coiffer, ils ont une brillance grasse désagréable et ont un toucher ciré également désagréable.
L'ampleur des conséquences de ces facteurs, presque tous inévitables, est très variable. Elle dépend par exemple de la qualité des cheveux, de leurs longueurs et de la coiffure adoptée.

Quoiqu'il en soit, pour lutter contre ces désagréments, on utilise des shampooings. En effet, le lavage avec des compositions détergentes est très efficace, il permet d'éliminer les salissures, les pellicules. Il est alors possible, au séchage, de replacer la chevelure dans la forme désirée. Cependant, l'effet bénéfique du shampooing s'estompe et on retrouve en quelques jours les problèmes décrits plus haut. En conséquence, on a tendance à augmenter la fréquence des shampooings.
Pour effectuer un shampooing, il faut avoir une source d'eau, de préférence chaude ou tiède.
Les compositions de shampooing sont à base de quantités importantes de tensioactifs qui peuvent générer des désagréments tels que des picotements au niveau du cuir chevelu ou des yeux.

Pour nettoyer plus rapidement les cheveux et éviter de mouiller les cheveux, on a déjà proposer d'utiliser des shampooings dits "secs". Cette technique consiste à pulvériser des particules absorbantes sur les cheveux puis à brosser activement la chevelure pour éliminer les particules. Mais en général, l'élimination complète des particules est très difficile à obtenir. Les résultats ne sont pas très satisfaisants : la brillance des cheveux est faible et leur toucher est rêche.

La présente invention a notamment pour but de résoudre les problèmes ci-dessus.

Plus précisément encore, la présente invention vise à proposer un procédé de nettoyage des cheveux qui conduise, en fin d'opération, à des cheveux présentant un toucher et un aspect visuel neutres.

Or, à la suite d'importantes recherches menées sur la question, il a maintenant été trouvé par la Demanderesse, et ceci de façon tout à fait inattendue et surprenante, que ce but, et d'autres, pouvaient être atteints en mettant en oeuvre des compositions cosmétiques liquides notamment capillaire, destinées au nettoyage des cheveux comprenant dans un milieu aqueux cosmétiquement acceptable :
- de 10 à 90% en poids par rapport au poids total de la composition d'au moins un monoalcool en C2-C4,
- de 0,01 à 5% en poids par rapport au poids total de la composition d'au moins un agent tensioactif choisi parmi les tensioactifs anioniques, les tensioactifs amphotères et/ou zwittérioniques et les tensioactifs non ioniques,
- de 0,01 à 5% en poids par rapport au poids total de la composition d'un agent solubilisant les composés liposolubles différent des tensioactifs et choisi parmi les hydrocarbures linéaires ou ramifiés, les polyoléfines hydrogénées ou non hydrogénées, les silicones volatiles et leurs mélanges,
- de 1 à 89,98 % en poids d'eau par rapport au poids total de la composition.

Les cheveux nettoyés avec les compositions selon l'invention possèdent les caractéristiques des cheveux propres : le toucher est naturel, les cheveux sont brillants (l'aspect gras a disparu), la chevelure est aérée, légère et il n'y a pas de pellicules. De plus les cheveux sont plus disciplinés, plus souples, plus lisses. D'autre part, le traitement retire les mauvaises odeurs.

Aussi, l'invention a encore pour objet l'utilisation cosmétique d'une composition cosmétique telle que définie ci-dessus pour le nettoyage des cheveux.

La présente invention a également pour objet un procédé de nettoyage des cheveux, ledit procédé étant caractérisé par le fait qu'on applique sur les cheveux secs une composition telle que définie ci-dessus, puis que l'on sèche les cheveux.

Les monoalcools en C2-C4 sont linéaires ou ramifiés. Ils sont notamment choisis parmi l'éthanol, l'isopropanol, le tertio-butanol.

Les alcools sont de préférence présent dans des concentrations allant de10 à 50% en poids et plus particulièrement de 10 à 25% en poids par rapport au poids total de la composition.

Comme tensioactifs non ioniques, on peut utiliser de préférence les alkylpolyglycosides et notamment les alkylpolyglucosides (APG) ayant un groupe alkyle comportant de 6 à 30 atomes de carbone (alkyl-C6-C30 polyglucosides) et de préférence 8 à 16 atomes de carbone comme par exemple le decylglucoside (Alkyl-C9/C11-polyglucoside (1.4) tel que le produit commercialisé sous la dénomination MYDOL 10 par la société Kao Chemicals, le produit commercialisé sous la dénomination PLANTAREN 2000 UP ou PLANTACARE 2000 UP par la société Henkel, et le produit commercialisé sous la dénomination ORAMIX NS 10 par la société Seppic ; le caprylyl/capryl glucoside comme le produit commercialisé sous la dénomination ORAMIX CG 110 par la Société Seppic ; le laurylglucoside comme les produits commercialisés sous les dénominations PLANTAREN 1200 N et PLANTACARE 1200 par la société Henkel ; et le coco-glucoside comme le produit commercialisé sous la dénomination PLANTACARE 818/UP par la société Henkel ;
Comme tensioactifs anioniques, on peut utiliser par exemple les carboxylates, les dérivés des aminoacides, les alkyl sulfates, les alkyl éther sulfates, les sulfonates, les iséthionates, les taurates, les sulfosuccinates, les alkylsulfoacétates, les phosphates et alkylphosphates, les polypeptides, les dérivés anioniques d'alkyl polyglucoside, les savons d'acides gras, et leurs mélanges.

Comme carboxylates, on peut citer par exemple les sels (par exemple alcalins) de N-acylaminoacides ; les alkyl(C8-C20)amidoéthercarboxylates (AEC) comme le lauryl amidoether carboxylate de sodium (3 OE) commercialisé sous la dénomination AKYPO FOAM 30 par la société Kao Chemicals ; les alkyl(C8-C20)éthercarboxylates, comme le lauryl ether carboxylate de sodium (C12-14-16 65/25/10) oxyéthyléné (6 OE) commercialisé sous la dénomination AKYPO SOFT 45 NV par la société Kao Chemicals, l'acide lauryléther carboxylique oxyéthyléné à 4,5 OE (AKYPO RLM 45 de KAO); les acides gras d'huile d'olive polyoxyéthylénés et de carboxymethyl, comme le produit commercialisé sous la dénomination OLIVEM 400 par la société Biologia E Tecnologia ; le tri-decyl éther carboxylate de sodium oxyéthyléné (6 OE) commercialisé sous la dénomination NIKKOL ECTD-6NEX par la société Nikkol ; le 2-(2-Hydroxyalkyloxy) acétate de sodium commercialisé sous la dénomination BEAULIGHT SHAA par la société SANYO. On utilise de préférence les acides d'alkyl(C8-C20)amidoéthercarboxyliques et les acides d'alkyl(C8-C20)éthercarboxyliques et leurs sels.

Les dérivés des aminoacides peuvent être choisis par exemple parmi les sarcosinates et notamment les acylsarcosinates comme le lauroylsarcosinate de sodium commercialisé sous la dénomination SARKOSYL NL 97 par la société Ciba ou commercialisé sous la dénomination ORAMIX L 30 par la société Seppic, le myristoyl sarcosinate de sodium, commercialisé sous la dénomination NIKKOL SARCOSINATE MN par la société Nikkol, le palmitoyl sarcosinate de sodium, commercialisé sous la dénomination NIKKOL SARCOSINATE PN par la société Nikkol ; les alaninates comme le N-lauroyl-N-methylamidopropionate de sodium commercialisé sous la dénomination SODIUM NIKKOL ALANINATE LN 30 par la société Nikkol ou commercialisé sous la dénomination ALANONE ALE par la société Kawaken, et le N-lauroyl N-methylalanine triéthanolamine, commercialisé sous la dénomination ALANONE ALTA par la société Kawaken ; les N-acylglutamates comme le mono-cocoylglutamate de triéthanolamine commercialisé sous la dénomination ACYLGLUTAMATE CT-12 par la société Ajinomoto, et le lauroyl-glutamate de triéthanolamine commercialisé sous la dénomination ACYLGLUTAMATE LT-12 par la société Ajinomoto ; les aspartates comme le mélange de N-lauroylaspartate de triéthanolamine et de N-myristoylaspartate de triéthanolamine, commercialisé sous la dénomination ASPARACK LM-TS2 par la société Mitsubishi ; les citrates, et leurs mélanges.

Comme dérivés de la glycine, on peut citer le N-cocoylglycinate de sodium et le N-cocoylglycinate de potassium comme les produits commercialisés sous les dénominations AMILITE GCS-12 et AMILITE GCK-12 par la société Ajinomoto.

Comme alkyl éther sulfates, on peut citer par exemple le lauryl éther sulfate de sodium (C12-14 70/30) (2,2 OE) commercialisé sous les dénominations SIPON AOS 225 ou TEXAPON N702 PATE par la société Henkel, le lauryl éther sulfate d'ammonium (C12-14 70/30) (3 OE) commercialisé sous la dénomination SIPON LEA 370 par la société Henkel, l'alkyl (C12-C14) éther (9 OE) sulfate d'ammonium commercialisé sous la dénomination RHODAPEX AB/20 par la société Rhodia Chimie.

Comme sulfonates, on peut citer par exemple les alpha-oléfines sulfonates comme l'alpha-oléfine sulfonate de sodium (C14-16) commercialisé sous la dénomination BIO-TERGE AS-40 par la société Stepan, commercialisé sous les dénominations WITCONATE AOS PROTEGE et SULFRAMINE AOS PH 12 par la société Witco ou commercialisé sous la dénomination BIO-TERGE AS-40 CG par la société Stepan, l'oléfine sulfonate de sodium secondaire commercialisé sous la dénomination HOSTAPUR SAS 30 par la société Clariant ; les alkyl aryl sulfonates linéaires comme le xylène sulfonate de sodium commercialisé sous les dénominations MANROSOL SXS30, MANROSOL SXS40, MANROSOL SXS93 par la société Manro.

Comme iséthionates, on peut citer les acyliséthionates comme le cocoyl-iséthionate de sodium, tel que le produit commercialisé sous la dénomination JORDAPON CI P par la société Jordan.

Comme taurates, on peut citer le sel de sodium de méthyltaurate d'huile de palmiste commercialisé sous la dénomination HOSTAPON CT PATE par la société Clariant ; les N-acyl N-méthyltaurates comme le N-cocoyl N-methyltaurate de sodium commercialisé sous la dénomination HOSTAPON LT-SF par la société Clariant ou commercialisé sous la dénomination NIKKOL CMT-30-T par la société Nikkol, le palmitoyl methyltaurate de sodium commercialisé sous la dénomination NIKKOL PMT par la société Nikkol.

Comme sulfosuccinates, on peut citer par exemple le mono-sulfosuccinate d'alcool laurylique (C12/C14 70/30) oxyéthyléné (3 OE) commercialisé sous les dénominations SETACIN 103 SPECIAL, REWOPOL SB-FA 30 K 4 par la société Witco, le sel di-sodique d'un hemi-sulfosuccinate des alcools C12-C14, commercialisé sous la dénomination SETACIN F SPECIAL PASTE par la société Zschimmer Schwarz, l'oléamidosulfosuccinate di-sodique oxyéthyléné (2 OE) commercialisé sous la dénomination STANDAPOL SH 135 par la société Henkel, le mono-sulfosuccinate d'amide laurique oxyéthyléné (5 OE) commercialisé sous la dénomination LEBON A-5000 par la société Sanyo, le sel di-sodique de mono-sulfosuccinate de lauryl citrate oxyéthyléné (10 OE) commercialisé sous la dénomination REWOPOL SB CS 50 par la société Witco, le mono-sulfosuccinate de mono-éthanolamide ricinoléique commercialisé sous la dénomination REWODERM S 1333 par la société Witco.

Comme phosphates et alkylphosphates, on peut citer par exemple les monoalkylphosphates et les dialkylphosphates, tels que le mono-phosphate de lauryle commercialisé sous la dénomination MAP 20 par la société Kao Chemicals, le sel de potassium de l'acide dodécyl-phosphorique, mélange de mono- et di-ester (diester majoritaire) commercialisé sous la dénomination CRAFOL AP-31 par la société Cognis, le mélange de monoester et de di-ester d'acide octylphosphorique, commercialisé sous la dénomination CRAFOL AP-20 par la société Cognis, le mélange de monoester et de diester d'acide phophorique de 2-butyloctanol éthoxylé (7 moles d'OE), commercialisé sous la dénomination ISOFOL 12 7 EO-PHOSPHATE ESTER par la société Condea, le sel de potassium ou de triéthanolamine de mono-alkyl (C12-C13) phosphate commercialisé sous les références ARLATONE MAP 230K-40 et ARLATONE MAP 230T-60 par la société Uniqema, le lauryl phosphate de potassium commercialisé sous la dénomination DERMALCARE MAP XC-99/09 par la société Rhodia Chimie.

Les polypeptides sont obtenus par exemple par condensation d'une chaîne grasse sur les aminoacides de céréale et notamment du blé et de l'avoine. Comme polypeptides, on peut citer par exemple le sel de potassium de la lauroyl protéine de blé hydrolysée, commercialisé sous la dénomination AMINOFOAM W OR par la société Croda, le sel de triéthanolamine de cocoyl protéine de soja hydrolysée, commercialisé sous la dénomination MAY-TEIN SY par la société Maybrook, le sel de sodium des lauroyl amino-acides d'avoine, commercialisé sous la dénomination PROTEOL OAT par la société Seppic, l'hydrolysat de collagène greffé sur l'acide gras de coprah, commercialisé sous la dénomination GELIDERM 3000 par la société Deutsche Gelatine, les protéines de soja acylées par des acides de coprah hydrogénés, commercialisées sous la dénomination PROTEOL VS 22 par la société Seppic.

Les dérivés anioniques d'alkyl-polyglucosides peuvent être notamment des citrates, tartrates, sulfosuccinates, carbonates et éthers de glycérol obtenus à partir des alkyl polyglucosides. On peut citer par exemple le sel de sodium d'ester tartrique de cocoylpolyglucoside (1,4), commercialisé sous la dénomination EUCAROL AGE-ET par la société Cesalpinia, le sel di-sodique d'ester sulfosuccinique de cocoylpolyglucoside (1,4), commercialisé sous la dénomination ESSAI 512 MP par la société Seppic, le sel de sodium d'ester citrique de cocoyl polyglucoside (1,4) commercialisé sous la dénomination EUCAROL AGE-EC® par la société Cesalpinia. Un autre dérivé d'holoside anionique peut être le dodecyl-D-galactoside uronate de sodium commercialisé sous la dénomination DODECYL-D-GALACTOSIDE URONATE DE SODIUM par la société SOLIANCE.

Comme tensioactifs amphotères et zwitterioniques, on peut citer par exemple les alkyl (C8-C20)bétaïnes, les N-alkyl(C8-C20)amidobétaïnes et leurs dérivés, les dérivés de la glycine, les sultaïnes, les alkyl(C8-C20) polyaminocarboxylates, les alkyl(C8-C20)amphoacétates et leurs mélanges.

Comme bétaïnes, on peut citer par exemple la cocobétaïne comme le produit commercialisé sous la dénomination DEHYTON AB-30 par la société Henkel, la laurylbétaïne comme le produit commercialisé sous la dénomination GENAGEN KB par la société Clariant, la laurylbétaïne oxyethylénée (10 OE), comme le produit commercialisé sous la dénomination LAURYLETHER(10 OE)BETAINE par la société Shin Nihon Rica, la stéarylbétaïne oxyéthylénée (10 OE) comme le produit commercialisé sous la dénomination STEARYLETHER(10 OE)BETAINE par la société Shin Nihon Rica.

Parmi les N-alkylamidobétaines et leurs dérivés, on peut citer par exemple la cocamidopropyl bétaine commercialisée sous la dénomination LEBON 2000 HG par la société Sanyo, ou commercialisée sous la dénomination EMPIGEN BB par la société Albright & Wilson, la lauramidopropyl bétaïne commercialisée sous la dénomination REWOTERIC AMB12P par la société Witco.

Comme sultaïnes, on peut citer le cocoyl-amidopropylhydroxy-sulfobetaine commercialisé sous la dénomination CROSULTAINE C-50 par la société Croda.

Comme alkyl polyaminocarboxylates (APAC), on peut citer le cocoylpolyaminocarboxylate de sodium, commercialisé sous la dénomination AMPHOLAK 7 CX/C,et AMPHOLAK 7 CX par la société Akzo Nobel, le stéarylpolyamidocarboxylate de sodium commercialisé sous la dénomination AMPHOLAK 7 TX/C par la société Akzo Nobel, la carboxyméthyloléylpolypropylamine de sodium, commercialisé sous la dénomination AMPHOLAK XO7/C par la société Akzo Nobel.

Comme alkylamphoacétates, on peut citer par exemple le N-cocoyl-N-carboxyméthoxyéthyl-N-carboxyméthyl-éthylènediamine N-di-sodique (nom CTFA : disodium cocamphodiacetate) comme le produit commercialisé sous la dénomination MIRANOL C2M CONCENTRE NP par la société Rhodia Chimie, et le N-cocoyl-N-hydroxyéthyl-N-carboxyméthyl-éthylènediamine N-sodique (nom CTFA : sodium cocamphoacetate).

La composition peut comprendre aussi un mélange de ces tensioactifs.

Les agents tensioactifs sont de préférence choisis parmi les tensioactifs amphotères et/ou zwittérioniques, les alkylpolyglycosides et les tensioactifs anioniques ayant au moins un groupement carboxylate.

La nature et la concentration des différents tensioactifs sont choisis de telle sorte que la composition ne soit pas moussante lors de l'application sur les cheveux.

De préférence, les tensioactifs sont présents à des concentrations allant de 0,01 à 2% et plus particulèrement de 0,05 à 1% en poids par rapport au poids total de la composition et encore plus particulièrement de 0,075 à 0,5% en poids.

Les agents solubilisant les composés liposolubles ne sont pas des tensioactifs. Ces composés ont un HLB inférieur à 3 (HLB = Hydrophilic-Lipophilic Balance, au sens de Griffin ; voir J. Soc. Cosm. Chem. 1954 (vol 5), pp 249-256 ; équilibre entre le caractère hydrophile et le caractère lipophile).

Les agents solubilisant les composés liposolubles sont de préférence volatiles.

Par volatile, on entend au sens de la présente invention, tout composé ayant un point d'ébullition inférieur à 290°C à pression atmosphérique.

Les hydrocarbures ont de préférence de 8 à 16 atomes de carbones et sont de préférence ramifiés. Ce sont plus particulièrement les isoparaffines en C10-C14.

Les polyoléfines en particulier les poly-α-oléfines et plus particulièrement :
- de type polybutène, hydrogéné ou non, et de préférence polyisobutène, hydrogéné ou non.
   On utilise de préférence les oligomères d'isobutylène de poids moléculaire inférieur à 1000.
   A titre d'exemples de poly-α-oléfines utilisables dans le cadre de la présente invention, on peut plus particulièrement mentionner les polyisobutènes vendus sous le nom de PERMETHYL 99 A, 101 A , 102 A , 104 A (n=16) et 106 A (n=38) par la Société PRESPERSE Inc, ou bien encore les produits vendus sous le nom de ARLAMOL HD (n=3) par la Société ICI (n désignant le degré de polymérisation),
- de type polydécène, hydrogéné ou non. De tels produits sont vendus par exemple sous les dénominations ETHYLFLO par la société ETHYL CORP., et d'ARLAMOL PAO par la société ICI.

Les silicones volatiles sont plus particulièrement choisies parmi celles possédant un point d'ébullition compris entre 60° C et 260° C, et plus particulièrement encore parmi :
(i) les silicones cycliques comportant de 3 à 7 atomes de silicium et de préférence 4 à 5. Il s'agit, par exemple, de l'octaméthylcyclotétrasiloxane commercialisé notamment sous le nom de "VOLATILE SILICONE 7207" par UNION CARBIDE ou "SILBIONE 70045 V 2" par RHODIA CHIMIE, le décaméthylcyclopentasiloxane commercialisé sous le nom de "VOLATILE SILICONE 7158" par UNION CARBIDE, "SILBIONE 70045 V 5" par RHODIA CHIMIE, ainsi que leurs mélanges.
(ii) les silicones volatiles linéaires ayant 2 à 9 atomes de silicium et possédant une viscosité inférieure ou égale à 5.10⁻⁶m²/s à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, P. 27-32 - TODD & BYERS "Volatile Silicone fluids for cosmetics".

Les agents solubilisants des composés liposolubles sont de préférence présents à des concentreations allant de 0,1 à 3% en poids et plus particulièrement de 0,5 à 1,5% en poids par rapport au poids total de la composition.

La composition peut comprendre en outre les adjuvants classiquement mis en oeuvre dans les domaines considérés, comme par exemple les solvants organiques, les agents épaississants et gélifiants hydrophiles ou lipophiles, les adoucissants, les antioxydants, les opacifiants, les agents stabilisants, les chélateurs, les parfums, les filtres, les huiles essentielles, les matières colorantes, les pigments, les vitamines, les provitamines, les agents antipelliculaires, les actifs antiséborrhéiques qui permettent un nettoyage de l'excédent de sebum sur les cheveux, et les agents antimicrobiens qui éliminent des cheveux les microorganismes qui y sont éventuellement présents, les agents antichute des cheveux, les actifs favorisants la pousse des cheveux, les actifs hydrophiles ou lipophiles, les vésicules lipidiques encapsulant éventuellement un ou plusieurs actifs, ou tout autre ingrédient habituellement utilisé en cosmétique ou dermatologie. Elle peut éventuellement contenir aussi des conservateurs. Les quantités des différents constituants des compositions selon l'invention sont celles classiquement utilisées dans les domaines considérés. Bien entendu, ces adjuvants doivent être de nature et utilisés en quantité telle qu'ils ne perturbent pas le nettoyage des cheveux selon l'invention. La quantité de ces adjuvants peut aller par exemple de 0,01 à 30 % en poids par rapport au poids total de la composition.

La composition peut contenir en outre un additif choisi parmi les conservateurs, les agents de régulation de pH, les parfums.

La viscosité de la composition cosmétique selon l'invention est de préférence inférieure à 1500 mPa.s et, de préférence encore, inférieure à 1000 mPa.s. La viscosité est mesurée à la température ambiante (environ 25°C) avec un appareil RHEOMAT RM 180.

Le pH des compositions utilisées selon l'invention est généralement compris entre 2 et 12, de préférence de 3 à 8.

Les compositions selon l'invention comprennent de préférence de 40 à 89% en poids d'eau, plus particulièrement de 50 à 85% en poids et encore plus préférentiellement de 60 à 85% en poids, et préférentiellement de 70 à 85% en poids , par rapport au poids total de la composition.

Les compositions selon l'invention sont préparées selon les techniques bien connues de l'homme de l'art du domaine.

L'application de la composition peut se faire avec les mains, avec un embout applicateur, avec un vaporisateur, avec un peigne dispensateur et de préférence avec un substrat insoluble imprégné de la composition.

La demanderesse a trouvé que l'utilisation d'un substrat insoluble pour appliquer la composition donnait des résultats particulièrement intéressant.
Ainsi, selon un mode préféré de l'invention, les compositions selon l'invention peuvent servir aussi pour l'imprégnation de substrats insolubles dans l'eau pour constituer des articles, et notamment des lingettes ou des gants destinées au nettoyage des cheveux. Le substrat insoluble dans l'eau peut comprendre une ou plusieurs couches et il peut être choisi dans le groupe comprenant des matériaux tissés, des matériaux non-tissés, des mousses, des éponges, des ouates, en feuilles, boules ou films. Il peut s'agir notamment d'un substrat non tissé à base de fibres d'origine naturelle (lin, laine, coton, soie) ou d'origine synthétique (dérivés de cellulose, viscose, dérivés polyvinyliques, polyesters comme téréphtalate de polyéthylène, polyoléfines comme polyéthylène ou polypropylène, polyamides comme le Nylon, dérivés acryliques). Les non tissés sont décrits de façon générale dans RIEDEL « Nonwoven Bonding Methods & Materials », Nonwoven World (1987). Ces substrats sont obtenus selon les procédés usuels de la technique de préparation des non-tissés.

Quand le substrat est un non-tissé, on utilise de préférence un non-tissé épais, qui ne se met pas en boule et qui est assez solide pour ne pas se déliter et ne pas pelucher à l'application sur la peau. Il doit être absorbant, doux au moins sur une face pour le démaquillage des yeux en particulier. Comme non-tissés appropriés, on peut citer par exemple ceux commercialisés sous les dénominations Ultraloft 15285-01, Ultraloft 182-008, Ultraloft 182-010, Ultraloft 182-016 par la société BBA, Vilmed M1519 Blau, Vilmed M 1550 N et 112-132-3 par la société Freudenberg, celui commercialisé sous la dénomination Norafin 11601-010B par la société Jacob Holm Industries, celui commercialisé sous la dénomination Aquadim VE50 G1 NL (spun lace) par la société Tharreau, les non tissés flockés commercialisés sous les dénominations Univel 109 et Univel 119 par la société Uni Flockage.

Par ailleurs, ce substrat peut comporter une ou plusieurs couches ayant des propriétés identiques ou différentes et avoir des propriétés d'élasticité, de douceur et autres appropriées à l'usage recherché. Les substrats peuvent comporter par exemple deux parties ayant des propriétés d'élasticité différentes comme décrit dans le document WO-A-99/13861 ou comporter une seule couche avec des densités différentes comme décrit dans le document WO-A-99/25318 ou comporter deux couches de textures différentes comme décrit dans le document WO-A-98/18441.

Cet article constitue de manière préférée un gant mais peut aussi avoir une autre forme (lingette par exemple).

L'invention a donc aussi pour objet un article comprenant un substrat insoluble dans l'eau et une composition telle que définie ci-dessus, imprégnée sur le dite substrat.

L'invention a aussi pour objet l'utilisation de la composition telle que définie ci-dessus pour la préparation d'un article destiné au nettoyage des cheveux.

Selon un autre aspect de la présente invention on réalise un article cosmétique à usage unique comprenant au moins un substrat imprégné d'une composition cosmétique liquide telle que définie ci-dessus, ledit substrat étant perméable et comportant au moins un premier groupe de fibres et au moins un second groupe de fibres dont l'hydrophilie est différente de celle des fibres du premier groupe, lesdits premier et second groupes de fibres étant disposés de telle sorte qu'une première face du substrat présente une hydrophilie supérieure à l'hydrophilie d'une seconde face du substrat, opposée à la première, ladite première face étant externe au substrat de manière à pouvoir être mise au contact d'une surface à traiter, en particulier les cheveux.

Avec un tel article, le gain en efficacité d'un traitement cosmétique peut être amélioré de façon sensible en permettant une libération en plus grande quantité sur la surface à traiter, de la composition dont est imprégné l'article.

Par "perméable" on désigne un substrat apte à être traversé sur toute son épaisseur par ladite composition cosmétique. Ainsi, à l'inverse de certains articles de l'art antérieur auxquels il a été fait référence précédemment, l'article selon l'invention ne comporte pas de couche imperméable susceptible de former barrière à la composition qui l'imprègne.

Bien entendu, le substrat est insoluble dans l'eau, c'est à dire que son intégrité n'est pas affectée de manière sensible par la présence de la composition cosmétique aqueuse qui l'imprègne, et ce pendant au moins toute la durée de vie du produit.

Avantageusement, le substrat est monocouche. Par "substrat monocouche" on désigne un substrat où toutes les fibres qui le composent sont liées entre elles lors d'un même process de liage. Au final, dans l'épaisseur du substrat, la distribution des deux types de fibres est inhomogène et pourra être selon un profil caractérisé par :
- une majorité de fibres du premier groupe sur la première face du substrat ;
- une majorité de fibres du second groupe sur la seconde face du substrat et, entre les deux faces,
- une quantité relative des fibres du premier groupe variant selon un profil relativement progressif dont la pente est de signe opposé à celle du profil relativement progressif correspondant à la quantité relative de fibres du second groupe.

Une telle structure se différencie des structures multicouches dans lesquelles différentes couches sont formées et liées séparément, puis collées entre elles par thermocollage, soudure à chaud ou par ultrasons.

Avantageusement, la première face du substrat a un indice de relarguage sous pression statique (IRSPS1), la deuxième face du substrat ayant un indice de relarguage sous pression statique (IRSPS2), le rapport IRSPS1/IRSPS2 étant supérieur ou égal à 1,5.

### INDICE DE RELARGUAGE SOUS PRESSION STATIQUE (IRSPS) :

Le test qui va être décrit ci-après permet de mesurer l'indice de relarguage sous pression statique (IRSPS) des deux faces d'un article imprégné d'un produit cosmétique tel que défini précédemment. L'IRSPS détermine la quantité de liquide imprégné dans le support susceptible d'être libéré par chacune des deux faces du support sous l'effet d'une pression statique, et de voir par là s'il y a une différence de relarguage entre les 2 faces.

La gestuelle habituellement adoptée par la consommatrice lors de l'utilisation de l'article consistant à essuyer sa peau ou ses cheveux avec une première face de l'article, l'autre face dudit article étant en contact avec sa main, il est souhaitable de déterminer la quantité de liquide libérée simultanément par les deux faces du support.

A cet effet, on pèse précisément au centième de gramme près, au moyen d'une balance Mettler Toledo PR5002, deux feuilles de matériau absorbant de type papier essuie-tout, de marque Wypall L30 (référence 7303) fourni par Kimberley-Clark, dont le grammage est 50 g/m². Les feuilles de matériau absorbant doivent être au moins aussi grandes que l'article à tester et, de préférence, un peu plus grandes de sorte qu'elles dépassent du bord extérieur du support d'au moins deux centimètres.

On place la première feuille sur une plaque en verre de dimensions au moins aussi grandes que celles de l'article à tester. On pose l'article à tester sur la première feuille de Wypall L30. On pose par dessus la deuxième feuille de Wypall L30.

On pose sur l'ensemble une plaque métallique de surface au moins égale à la surface de l'article à tester, d'un poids de 3,7 kg. Après une minute on enlève la plaque et on pèse chaque feuille de Wypall L30.

Le poids de liquide qui a imprégné chaque feuille de Wypall L30 est calculé par différence de la masse du Wypall L30 après et avant le test. Ce poids de liquide correspond à la quantité de liquide qui a été libérée par chacune des deux faces du support, notée respectivement IRSPS1 pour la face ayant la plus grande hydrophilie et IRSPS2 pour la face ayant la plus faible hydrophilie.

Le rapport entre IRSPS1 et IRSPS2 indique la différence de relarguage entre les 2 faces de la lingette.

La valeur retenue pour le rapport IRSPS1/IRSPS2 est la valeur moyenne de mesures opérées sur quatre articles.

Avantageusement, le rapport IRSPS1/IRSPS2 va de 1,5 à 15, et de préférence, de 2,5 à 10, et de préférence encore, de 2,5 à 7.

Bien évidemment, lorsque l'article est creux et est configuré notamment sous forme d'un gant, on sépare les deux feuilles qui le constituent et on effectue le test sur les feuilles ainsi séparées.

### LE SUBSTRAT:

Le substrat est fabriqué de préférence selon le procédé de liage par jets d'eau, classiquement utilisés pour la préparation des non-tissés.

La première étape est la constitution des nappes de fibres ayant des hydrophilies différentes. Ces nappes peuvent être préparées selon différentes méthodes connues de l'homme de l'art, soit par cardage à partir de balles de fibres, soit par extrusion de polymère sous la forme de filaments continus de fibres, soit par voie aéro-pneumatique.

La nappe de fibres les plus hydrophiles ainsi que la nappe de fibres les moins hydrophiles sont déposées sur un tapis roulant et acheminées sous des rampes de jets d'eau à haute pression qui lient les fibres entre elles. Ces jets d'eau sous haute pression provoquent un réarrangement des fibres dans les trois directions au sein de la structure fibreuse conduisant ainsi à un enchevêtrement des fibres les liant entre elles sans ajout de liants chimiques. Généralement les nappes de fibres passent sous plusieurs rampes de jets d'eau dont la pression augmente graduellement de la première rampe jusqu'à la dernière. Cette technique est connue sous le nom d'hydroliage.

Le substrat peut par ailleurs être embossé, ajouré, calandré, imprimé ou subir tout type de traitement en fin de ligne. Ainsi, avantageusement, une desdites faces du support, notamment la moins hydrophile, est rendue exfoliante, en particulier par un calandrage à chaud.

Selon ce process particulier, Il est à noter que, bien que formé à partir de deux nappes de fibres, au final, le substrat ne comporte qu'une seule couche. Les risques de délaminage qui ont pu être observés dans des structures multicouches auxquelles il a été fait référence précédemment sont supprimés.

Les fibres les plus hydrophiles peuvent être choisies parmi les fibres de coton, de cellulose, ou de viscose.

Les fibres les moins hydrophiles peuvent être choisies parmi les fibres de polypropylène, polyester, polyamide, ou polyéthylène.

Selon un autre aspect de la présente invention on réalise un article cosmétique à usage unique comprenant au moins un substrat imprégné d'une composition cosmétique liquide, ledit substrat étant perméable et comportant au moins un premier groupe de fibres et au moins un second groupe de fibres dont l'hydrophilie est différente de celle des fibres du premier groupe, lesdits premier et second groupes de fibres étant disposés de telle sorte qu'une première face du substrat, externe à ce dernier, présente une hydrophilie supérieure à l'hydrophilie d'une seconde face du substrat, opposée à la première, ledit article comportant au moins une portion creuse ou concave, ladite portion creuse ou concave étant destinée :
i) soit à recevoir une surface à traiter de manière à mettre cette dernière en contact avec une surface interne de l'article ;
ii) soit à recevoir tout ou partie d'une main, en vue d'amener une surface externe de l'article en engagement avec une surface à traiter.

Un tel article creux ou concave peut être configuré sous forme d'un gant, d'une moufle, d'un chausson, d'un doigtier, d'un bonnet, d'une coiffe ou d'une charlotte.

Un tel article creux peut être obtenu par assemblage le long de leurs bords respectifs d'au moins "deux feuilles", ou par repliement d'une feuille sur elle même puis par fixation sur eux mêmes des bords de la feuille ainsi repliée.

Lorsque la portion creuse ou concave est destinée à recevoir tout ou partie d'une main, en vue d'amener une surface externe de l'article en engagement avec une surface à traiter, la face la plus hydrophile est à l'extérieur de la portion creuse ou concave.

Avantageusement, le substrat est imprégné par ladite composition cosmétique selon un taux allant de 100% à 1000%, de préférence, de 150% et 800%, et de préférence encore, de 150% et 400% en poids de composition par poids de substrat non imprégné.

Les exemples qui suivent permettront de mieux comprendre l'invention, sans toutefois présenter un caractère limitatif. Les quantités indiquées sont en % en poids, sauf mention contraire.

Dans les exemples qui suivent, donnés à titre illustratif et non limitatif, on donne des compositions concrètes conformes à l'invention. (MA signifie matière active)

### EXEMPLE 1 : Gant Nettoyant capillaire :

La composition nettoyante décrite ci-dessous est imprégnée à 200% sur un non-tissé Sandler ref. 03/02/1001.

| **Ingrédients :** | **% massique :** |
|---|---|
| Eau | Qsp 100% |
| Ethanol | 16 % MA |
| Disodium cocoamphodiacétate (MIRANOL C2M CONC de RHODIA CHIMIE) | 0,2 % MA |
| Isoparaffine C11-C12 (ISOPAR H de EXXON MOBIL CHEMICAL) | 1 % |
| Parfum, conservateur | qs |
| Agent de pH qs | pH 7 |

Le non-tissé Sandler ref. 03/02/1001 est obtenu par hydroliage et composé de 100% de fibres de viscose sur une face et de 100% de fibres de Polypropylene (PP) sur l'autre face. Il est découpé à la forme d'une moufle ou d'un gant dont les faces PP sont soudées vers l'intérieur par les bords de façon à délimiter une poche dans laquelle introduire la main.

Sur des cheveux lavés depuis quelques jours, on masse une demi-tête avec un gant comprenant la composition selon l'invention. On masse les cheveux pendant environ 30 secondes puis on laisse sécher les cheveux à l'air libre.

Après séchage, du côté gauche (invention), la chevelure est plus nette, plus aérée qu'à droite sans traitement. Le toucher est également plus agréable à gauche (invention) qu'avant traitement.

Les cheveux sont plus lisses au toucher, plus légers et plus souples. Ils ont plus de volume et la mise en forme est plus facile.

### EXEMPLE 2 : Gant Nettoyant capillaire :

La composition nettoyante décrite ci-dessous est imprégnée à 200% sur un non-tissé Sandler ref. 03/02/1001.

| **Ingrédients :** | **% massique :** |
|---|---|
| Eau | Qsp 100 |
| Ethanol dénaturé. (93.1 % MA dans l'eau) | 20 % |
| Disodium cocoamphodiacétate (MIRANOL C2M CONC de RHODIA CHIMIE) | 0,2 % MA |
| Polyisobutène hydrogéné | 5 % |
| Parfum | qs |
| Agent de pH qs | pH 7 |

Le non-tissé Sandler ref. 03/02/1001 est obtenu par hydroliage et composé de 100% de fibres de viscose sur une face et de 100% de fibres de Polypropylene (PP) sur l'autre face. Il est découpé à la forme d'une moufle ou d'un gant dont les faces PP sont soudées vers l'intérieur par les bords de façon à délimiter une poche dans laquelle introduire la main.

### EXEMPLE 3 : Gant Nettoyant capillaire :

La composition nettoyante décrite ci-dessous est imprégnée à 200% sur un non-tissé Sandler ref. 03/02/1001.

| **Ingrédients :** | **% massique :** |
|---|---|
| Eau | Qsp 100 |
| Ethanol | 16 % |
| Acide Lauryléthercarboxylique à 4,5 moles d'oxyde d'éthylène (AKYPO RLM 45 de KAO) | 0,4 % MA |
| Isoparaffine C11-C12 (ISOPAR H de EXXON MOBIL CHEMICAL) | 1 % |
| Parfum, conservateur | qs |
| Agent de pH qs | pH 7 |

Les cheveux sont plus lisses au toucher, plus légers et plus souples. Ils ont plus de volume et la mise en forme est plus facile.

## Revendications

1. Composition cosmétique capillaire, comprenant dans un milieu aqueux cosmétiquement acceptable :
- de 10 à 90% en poids par rapport au poids total de la composition d'au moins un monoalcool en C2-C4,
- de 0,01 à 5% en poids par rapport au poids total de la composition d'au moins un agent tensioactif choisi parmi les tensioactifs anioniques, les tensioactifs amphotères et/ou zwittérioniques et les tensioactifs non ioniques,
- de 0,01 à 5% en poids par rapport au poids total de la composition d'un agent solubilisant les composés liposolubles, ledit agent étant choisi parmi les hydrocarbures linéaires ou ramifiés, les polyoléfines hydrogénées ou non hydrogénées, les silicones volatiles et leurs mélanges,
- de 1 à 89,98 % en poids d'eau par rapport au poids total de la composition.

2. Composition selon la revendication 1, **caractérisée en ce que** les monoalcools en C2-C4 sont choisis parmi l'éthanol, l'isopropanol, le tertio-butanol.

3. Composition selon l'une quelconque des revendications précédentes, **caractérisée en ce que** le ou les monoalcools sont présents dans des concentrations allant de10 à 50% en poids et plus particulièrement de 10 à 25% en poids par rapport au poids total de la composition.

4. Composition selon l'une quelconque des revendications précédentes, **caractérisée en ce que** les agents tensioactifs sont choisis parmi les tensioactifs amphotères et/ou zwittérioniques, les alkylpolyglucosides et les tensioactifs anioniques ayant au moins un groupement carboxylate.

5. Composition selon l'une quelconque des revendications précédentes, **caractérisée en ce que** les agents tensioactifs amphotères et/ou zwittérioniques sont choisis parmi les alkylamphoacétates.

6. Composition selon l'une quelconque des revendications précédentes, **caractérisée en ce que** les agents tensioactifs anioniques sont choisis parmi les acides d'alkyl(C8-C20)amidoéthercarboxyliques et les acides d'alkyl(C8-C20)éthercarboxyliques et leurs sels.

7. Composition selon l'une quelconque des revendications précédentes, **caractérisée en ce que** les agents tensioactifs sont présents à des concentrations allant de 0,01 à 2% et plus particulèrement de 0,05 à 1% en poids par rapport au poids total de la composition et encore plus particulièrement de 0,1 à 0,5% en poids.

8. Composition selon l'une quelconque des revendications précédentes, **caractérisée en ce que** les agents solubilisants sont présents à des concentrations allant de 0,1 à 3% et plus particulèrement de 0,5 à 1,5% en poids par rapport au poids total de la composition.

9. Utilisation de la composition telle que définie à l'une quelconque des revendications 1 à 8 pour la préparation d'un article destiné au nettoyage des cheveux.

10. Article cosmétique à usage unique comprenant au moins un substrat imprégné d'une composition cosmétique liquide telle que définie à l'une quelconque des revendications 1 à 8.

11. Article cosmétique à usage unique comprenant au moins un substrat imprégné d'une composition cosmétique liquide telle que définie à l'une quelconque des revendications 1 à 8, ledit substrat étant perméable et comportant au moins un premier groupe de fibres et au moins un second groupe de fibres dont l'hydrophilie est différente de celle des fibres du premier groupe, lesdits premier et second groupes de fibres étant disposés de telle sorte qu'une première face du substrat présente une hydrophilie supérieure à l'hydrophilie d'une seconde face du substrat, opposée à la première, ladite première face étant externe au substrat de manière à pouvoir être mise au contact d'une surface à traiter, en particulier les cheveux.

12. Article cosmétique selon la revendication 11 **caractérisé en ce que** le substrat est monocouche.

13. Article cosmétique selon l'une quelconque des revendications 10 à 12 **caractérisé en ce que** le substrat est imprégné par ladite composition cosmétique selon un taux allant de 100% à 1000%, de préférence, de 150% et 800%, et de préférence encore, de 150% et 400% en poids de composition par poids de substrat non imprégné.

14. Utilisation d'une composition cosmétique selon l'une quelconque de revendications 1 à 8 pour le nettoyage des cheveux.

15. Procédé de nettoyage des cheveux, **caractérisé par le fait que** l'on applique sur les cheveux secs une composition telle décrite à l'une quelconque des revendications 1 à 8.

16. Procédé de nettoyage des cheveux, **caractérisé par le fait que** l'on applique sur les cheveux secs un article selon l'une quelconque des revendications 10 à 13.
